(19) Europäisches Patentamt
European Patent Office
Office européen des brevets

(11) Veröffentlichungsnummer: **0 303 906**
**A1**

(12) # EUROPÄISCHE PATENTANMELDUNG

(21) Anmeldenummer: 88112697.3

(22) Anmeldetag: 04.08.88

(51) Int. Cl.⁴: C07D 231/44 , C07D 231/22 , A01N 43/56

(30) Priorität: 15.08.87 DE 3727260

(43) Veröffentlichungstag der Anmeldung: 22.02.89 Patentblatt 89/08

(84) Benannte Vertragsstaaten: BE CH DE FR GB IT LI NL

(71) Anmelder: BAYER AG
D-5090 Leverkusen(DE)

(72) Erfinder: Schallner, Otto, Dr.
Noldeweg 22
D-4019 Monheim(DE)
Erfinder: Gallenkamp, Bernd, Dr.
Paul-Ehrlich-Strasse 13
D-5600 Wuppertal(DE)
Erfinder: Becker, Benedikt, Dr.
Metzkausener Strasse 14
D-4020 Mettmann(DE)
Erfinder: Behrenz, Wolfgang, Dr.
Untergründemich 14
D-5063 Overath(DE)
Erfinder: Andrews, Peter, Dr.
Gellertweg 2
D-5600 Wuppertal(DE)

(54) **1-Arylpyrazole.**

(57) Die Erfindung betrifft neue 1-Arylpyrazole der allgemeinen Formel (I),

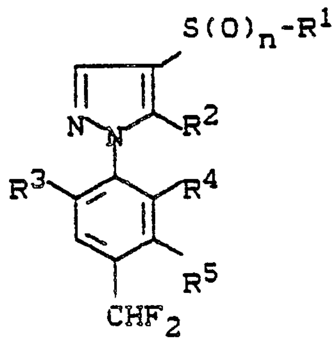

(I)

in welcher
$R^1$ für Alkyl oder Halogenalkyl steht,
$R^2$ für Wasserstoff, Halogen oder für einen Rest

EP 0 303 906 A1

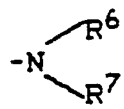

steht, in welchem
$R^6$ und $R^7$ unabhängig voneinander für Wassserstoff, Alkyl, Alkenyl oder Alkinyl stehen,
$R^3$ für Wasserstoff oder Halogen steht,
$R^4$ für Halogen steht,
$R^5$ für Wasserstoff oder Halogen steht und
n für eine Zahl 0, 1 oder 2 steht.
welche als Schädlingsbekämpfungsmittel verwendet werden können.

# 1-Arylpyrazole

Die Erfindung betrifft neue 1-Arylpyrazole, mehrere Verfahren zu ihrer Herstellung und ihre Verwendung als Schädlingsbekämpfungsmittel.

Es ist bereits bekannt, daß bestimmte 1-Arylpyrazole, wie beispielsweise das 1-(2,6-Dichlor-4-trifluormethylphenyl)-4-trifluormethylthiopyrazol oder das 5-Amino-1-(2,6-dichlor-4-trifluormethylphenyl)-4-dichlorfluormethylthio-pyrazol oder das 5-Amino-1-(2,6-dichlor-4-trifluormethylphenyl)-4-trifluormethylthio-pyrazol oder das 5-Amino-1-(2,6-dichlor-4-trifluormethylphenyl)-4-dichlorfluormethylsulfinyl-pyrazol oder das 5-Amino-1-(2,6-dichlor-4-trifluormethylphenyl)-4-dichlorfluormethylsulfonylpyrazol eine gute Wirkung gegen Schädlinge, insbesondere gegen Insekten besitzen (vergl. EP-A-201 852 sowie EP-A-161 102).

Die Wirkungshöhe bzw. Wirkungsdauer dieser vorbekannten Verbindungen ist jedoch, insbesondere bei bestimmten Insekten oder bei niedrigen Anwendungskonzentrationen nicht unter allen Anwendungsbedingungen völlig zufriedenstellend.

Es wurden neue 1-Arylpyrazole der allgemeinen Formel (I),

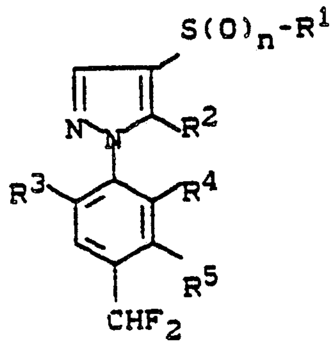

(I)

in welcher

$R^1$ für Alkyl oder Halogenalkyl steht,

$R^2$ für Wasserstoff, Halogen oder für einen Rest

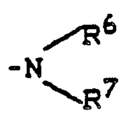

steht, in welchem

$R^6$ und $R^7$ unabhängig voneinander für Wasserstoff, Alkyl, Alkenyl oder Alkinyl stehen,

$R^3$ für Wasserstoff oder Halogen steht,

$R^4$ für Halogen steht,

$R^5$ für Wasserstoff oder Halogen steht und

n für eine Zahl 0, 1 oder 2 steht,

gefunden.

Weiterhin wurde gefunden, daß man die neuen 1-Arylpyrazole der allgemeinen Formel (I) erhält, wenn man

(a) zur Herstellung der 1-Arylpyrazole der Formel (Ia),

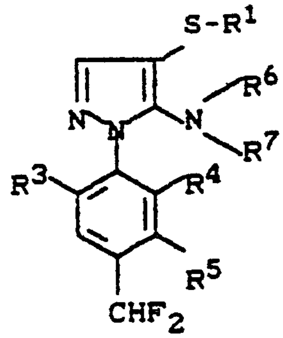

(Ia)

in welcher $R^1$, $R^3$, $R^4$, $R^5$, $R^6$ und $R^7$ die oben angegebene Bedeutung haben, 4-unsubstituierte 1-Arylpyrazole der Formel (II),

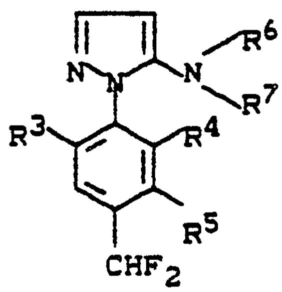

(II)

in welcher $R^3$, $R^4$, $R^5$, $R^6$ und $R^7$ die oben angegebene Bedeutung haben, mit Sulfenylchloriden der Formel (III),

$Cl\text{-}S\text{-}R^1$ (III)

in welcher $R^1$ die oben angegebene Bedeutung hat, gegebenenfalls in Gegenwart eines Verdünnungsmittels und gegebenenfalls in Gegenwart eines Säurebindemittels umsetzt; oder

b) zur Herstellung der 1-Arylpyrazole der Formel (Ib),

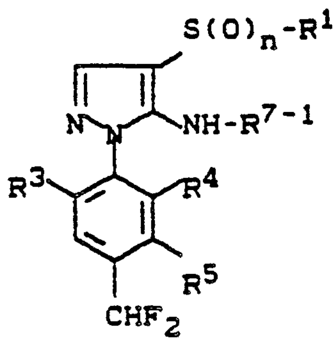

(Ib)

in welcher $R^1$, $R^3$, $R^4$, $R^5$ und n die oben angegebene Bedeutung haben und $R^{7-1}$ für Alkyl, Alkenyl oder Alkinyl steht, 5-(N-Acylamino)-1-arylpyrazole der Formel (IV),

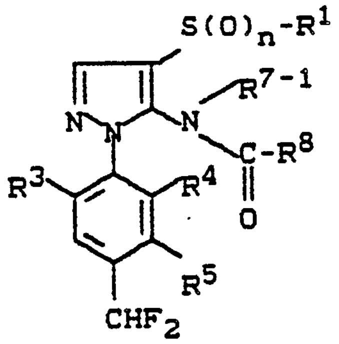

(IV)

in welcher
$R^1$, $R^3$, $R^4$, $R^5$, $R^{7-1}$ und n die oben angegebene Bedeutung haben und
$R^8$ für Alkyl steht,
mit Säuren als Katalysator gegebenenfalls in Gegenwart eines Verdünnungsmittels deacyliert; oder

(c) zur Herstellung der 1-Arylpyrazole der Formel (Ic),

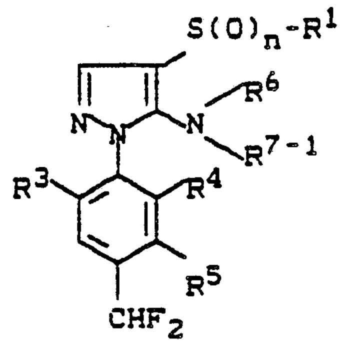

(Ic)

in welcher
$R^1$, $R^3$, $R^4$, $R^5$, $R^6$ und n die oben angegebene Bedeutung haben und
$R^{7-1}$ für Alkyl, Alkenyl oder Alkinyl steht,
1-Arylpyrazole der Formel (Iz)

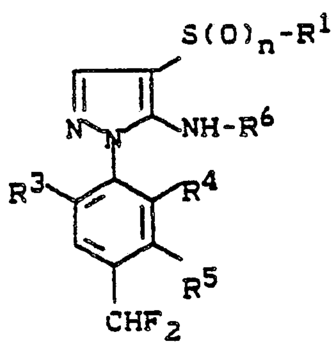

(Iz)

in welcher
$R^1$, $R^3$, $R^4$, $R^5$, $R^6$ und n die oben angegebene Bedeutung haben,
mit Alkylierungsmitteln der Formel (V),

$R^{7-1}$ - $A^1$ (V)

in welcher
$R^{7-1}$ die oben angegebene Bedeutung hat und
$A^1$ für eine elektronenanziehende Abgangsgruppe steht,

gegebenenfalls in Gegenwart eines Verdünnungsmittels und gegebenenfalls in Gegenwart eines Reaktionshilfsmittels umsetzt; oder

(d) zur Herstellung der 1-Arylpyrazole der Formel (Id),

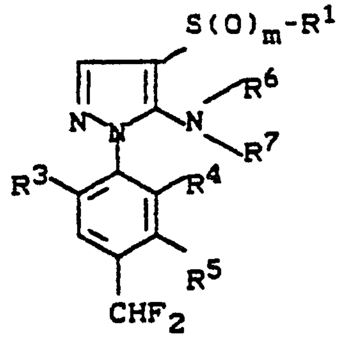

(Id)

in welcher
$R^1$, $R^3$, $R^4$, $R^5$, $R^6$ und $R^7$ die oben angegebene Bedeutung haben und
m für eine Zahl 1 oder 2 steht,
1-Arylpyrazole der Formel (Ia),

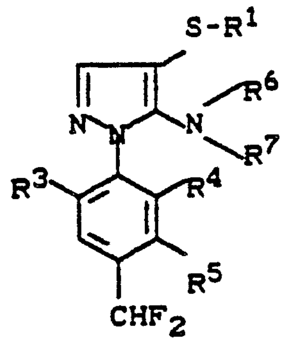

(Ia)

$R^1$, $R^3$, $R^4$, $R^5$, $R^6$ und $R^7$ die oben angegebene Bedeutung haben,
mit einem Oxidationsmittel, gegebenenfalls in Gegenwart eines Verdünnungsmittels, gegebenenfalls in Gegenwart eines Reaktionshilfsmittels und gegebenenfalls in Gegenwart eines Katalysators umsetzt; oder

(e) zur Herstellung der 1-Arylpyrazole der Formel (Ie),

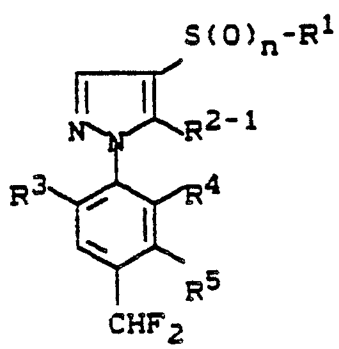

(Ie)

in welcher
$R^1$, $R^3$, $R^4$, $R^5$ und n die oben angegebene Bedeutung haben und
$R^{2-1}$ für Wasserstoff oder Halogen steht,
1-Arylpyrazole der Formel (Iy),

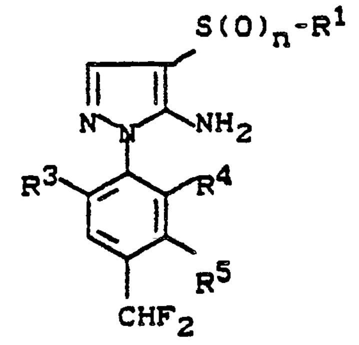

(Iy)

in welcher

$R^1$, $R^3$, $R^4$, $R^5$ und n die oben angegebene Bedeutung haben,

mit einem anorganischen oder organischen Nitrit in Gegenwart eines Reaktionshilfsmittels und gegebenenfalls in Gegenwart einer Halogenwasserstoffsäure sowie gegebenenfalls in Gegenwart eines Verdünnungsmittels umsetzt.

Schließlich wurde gefunden, daß die neuen 1-Arylpyrazole eine gute Wirkung gegen Schädlinge, insbesondere gegen Insekten aufweisen.

Überraschenderweise zeigen die erfindungsgemäßen 1-Arylpyrazole der allgemeinen Formel (I) eine erheblich bessere insektizide Wirksamkeit als die aus dem Stand der Technik bekannten 1-Arylpyrazole (wie beispielsweise das 1-(2,6-Dichlor-4-trifluormethylphenyl)-4-trifluormethylthiopyrazol oder das 5-Amino-1-(2,6-dichlor-4-trifluormethylphenyl)-4-dichlorfluormethylthio-pyrazol oder das 5-Amino-1-(2,6-dichlor-4-trifluormethylphenyl)-4-trifluormethylthio-pyrazol oder das 5-Amino-1-(2,6-dichlor-4-trifluormethylphenyl)-4-dichlorfluormethylsulfinyl-pyrazol oder das 5-Amino-1-(2,6-dichlor-4-trifluormethylphenyl)-4-dichlorfluorm ethylsulfonyl-pyrazol).

In den allgemeinen Formeln steht Alkyl für geradkettiges oder verzweigtes Alkyl mit vorzugsweise 1 bis 6, insbesondere 1 bis 4 Kohlenstoffatomen. Beispielhaft seien Methyl, Ethyl, n- und i-Propyl, n-, i- und t-Butyl genannt.

Halogenalkyl der allgemeinen Formeln ist geradkettig oder verzweigt und enthält vorzugsweise 1 bis 4, insbesondere 1 oder 2 Kohlenstoffatome und vorzugsweise 1 bis 5, insbesondere 1 bis 3 gleiche oder verschiedene Halogenatome, wobei als Halogenatome vorzugsweise Fluor, Chlor und Brom, insbesondere Fluor und Chlor stehen. Beispielhaft seien Trifluormethyl, Dichlorfluormethyl, Chlor-di-fluormethyl, Brommethyl, 2,2,2-Trifluorethyl und Pentafluorethyl, vorzugsweise Trifluormethyl und Dichlorfluormethyl, genannt.

In den allgemeinen Formeln bedeutet Alkenyl geradkettiges oder verzweigtes Alkenyl mit vorzugsweise 2 bis 6, insbesondere 2 bis 4 Kohlenstoffatomen. Beispielhaft seien Ethenyl, Propenyl-(1), Propenyl-(2) und Butenyl-(3) genannt.

Alkinyl bedeutet in den allgemeinen Formeln geradkettiges oder verzweigtes Alkinyl mit vorzugsweise 2 bis 6, insbesondere 2 bis 4 Kohlenstoffatomen. Beispielhaft seien gegebenenfalls substituiertes Ethinyl, Propinyl-(1), Propinyl-(2) und Butinyl-(3) genannt.

Halogen bedeutet in den allgemeinen Formeln (wenn nichts anderes angegeben ist) Fluor, Chlor, Brom und Jod, vorzugsweise Fluor, Chlor und Brom, besonders bevorzugt Fluor und Chlor und ganz besonders bevorzugt Chlor.

In den allgemeinen Formeln steht $R^1$ vorzugsweise für Halogenalkyl, vorzugsweise für Trifluormethyl und Dichlorfluormethyl.

$R^2$ steht vorzugsweise für Wasserstoff oder $-NR^6R^7$, wobei $R^6$ und $R^7$ vorzugsweise Wasserstoff bedeuten.

In den allgemeinen Formeln steht $R^3$ vorzugsweise für Halogen (besonders bevorzugt für Fluor oder Chlor, insbesondere für Chlor).

$R^5$ steht in den allgemeinen Formeln vorzugsweise für Wasserstoff.

Bevorzugt sind Verbindungen der Formel (I), in welchen

$R^1$ für Alkyl mit 1 bis 4 Kohlenstoffatomen oder für Halogenalkyl mit 1 bis 4 Kohlenstoffatomen und 1 bis 9 gleichen oder verschiedenen Halogenatomen steht,

$R^2$ für Wasserstoff, Fluor, Chlor, Brom oder für einen Rest

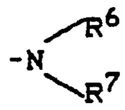

steht, in welchem
$R^6$ und $R^7$ unabhängig voneinander für Wasserstoff, Alkyl mit 1 bis 6 (vorzugsweise 1 bis 4) Kohlenstoffatomen, Alkenyl oder Alkinyl mit 2 bis 6 (vorzugsweise 2 bis 4) Kohlenstoffatomen stehen,
$R^3$ für Wasserstoff, Fluor, Chlor oder Brom steht,
$R^4$ für Fluor, Chlor oder Brom steht,
$R^5$ für Wasserstoff, Fluor, Chlor oder Brom steht und
n für eine Zahl 0, 1 oder 2 steht.

Besonders bevorzugt sind Verbindungen der Formel (I), in welchen
$R^1$ für Alkyl mit 1 bis 4 Kohlenstoffatomen oder für Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl oder Brompropyl steht,
$R^2$ für Wasserstoff, Fluor, Chlor, Brom oder einen Rest

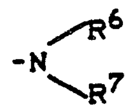

steht, in welchem
$R^6$ und $R^7$ unabhängig voneinander für Wasserstoff, Alkyl mit 1 bis 4 Kohlenstoffatomen, Allyl, Propargyl, Butenyl, Butinyl, Pentenyl oder Pentinyl stehen,
$R^3$ für Wasserstoff, Fluor, Chlor oder Brom steht,
$R^4$ für Fluor, Chlor oder Brom steht,
$R^5$ für Wasserstoff, Fluor, Chlor oder Brom steht, und
n für eine Zahl 0, 1 oder 2 steht.

Ganz besonders bevorzugt sind Verbindungen der Formel (I), in welchen
$R^1$ für Methyl, Ethyl, Trifluormethyl, Dichlorfluormethyl oder Difluorchlormethyl steht,
$R^2$ für Wasserstoff, Chlor, Brom oder einen Rest

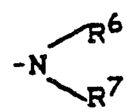

steht, in welchem
$R^6$ und $R^7$ unabhängig voneinander für Wasserstoff, Methyl, Ethyl oder Allyl stehen,
$R^3$ für Wasserstoff, Fluor oder Chlor steht,
$R^4$ für Fluor oder Chlor steht,
$R^5$ für Wasserstoff, Fluor oder Chlor steht und
n für eine Zahl 0, 1 oder 2 steht.

Speziell bevorzugt werden Verbindungen der Formel (I), in welchen
$R^1$ für Halogenalkyl mit 1 bis 4 (vorzugsweise 1 oder 2) Kohlenstoffatomen (vorzugsweise Trifluormethyl oder Dichlorfluormethyl) steht,
$R^2$ für Wasserstoff oder die $-NH_2$-Gruppe steht,
$R^3$ und $R^4$ für Halogen (vorzugsweise Chlor) stehen,
$R^5$ für Wasserstoff steht und
n 0, 1 oder 2 bedeutet.

Ganz speziell bevorzugt wurden Verbindungen der Formel (I), in welchen
$R^1$ für Trifluormethyl oder Dichlorfluormethyl stehen,
$R^2$ für Wasserstoff oder $-NH_2$ steht,

$R^3$ und $R^4$ für Chlor stehen,
$R^5$ für Wasserstoff steht, und
n für 0, 1 oder 2 steht.

Die im Zusammenhang mit den Verbindungen der Formel (I) aufgeführten allgemeinen und bevorzugten Definitionen gelten auch für die entsprechenden Definitionen in den jeweiligen Ausgangs- und Zwischenprodukten.

Verwendet man beispielsweise 5-Amino-1-(2,6-dichlor-4-difluormethylphenyl)-pyrazol und Trifluormethansulfenylchlorid als Ausgangsstoffe, so läßt sich der Reaktionsablauf der erfindungsgemäßen Verfahrensvariante (a) durch das folgende Formelschema darstellen:

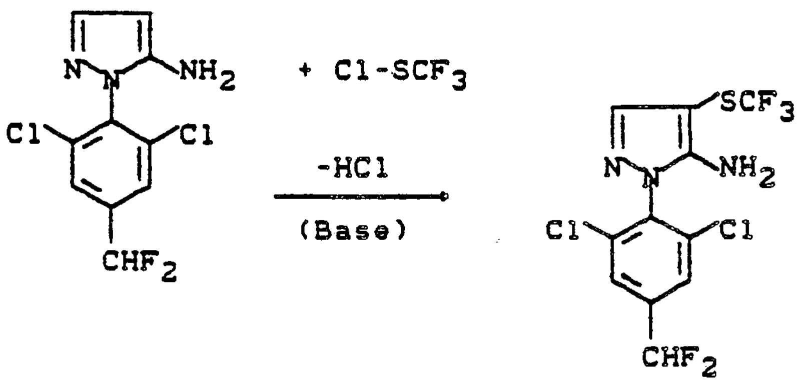


Verwendet man beispielsweise 5-(N-Ethyl-acetamido)-1-(2,4-dichlor-4-difluormethylphenyl)-4-dichlorfluormethylthio-pyrazol als Ausgangsverbindung, so läßt sich der Reaktionsablauf der erfindungsgemäßen Verfahrensvariante (b) durch das folgende Formelschema darstellen:

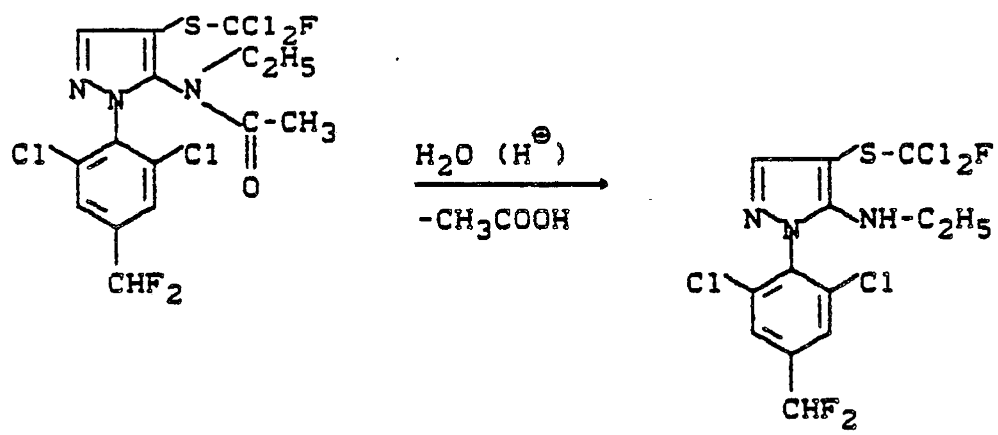


Verwendet man beispielsweise 5-Amino-1-(2,3,6-trichlor-4-difluormethylphenyl)-4-trifluormethylthio-pyrazol und Dimethylsulfat als Ausgangsstoffe, so läßt sich der Reaktionsablauf der erfindungsgemäßen Verfahrensvariante (c) durch das folgende Formelschema darstellen:

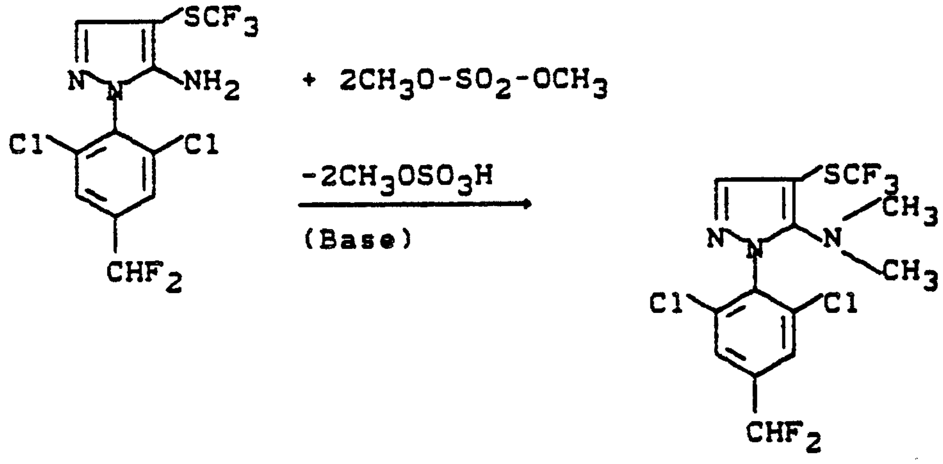


Verwendet man beispielsweise 5-Amino-1-(2,6-dichlor-4-difluormethylphenyl)-4-trifluormethylthiopyrazol als Ausgangsverbindungen und m-Chlorperbenzoesäure als Oxidationsmittel, so läßt sich der Reaktionsablauf der erfindungsgemäßen Verfahrensvariante (d) durch das folgende Formelschema darstellen:

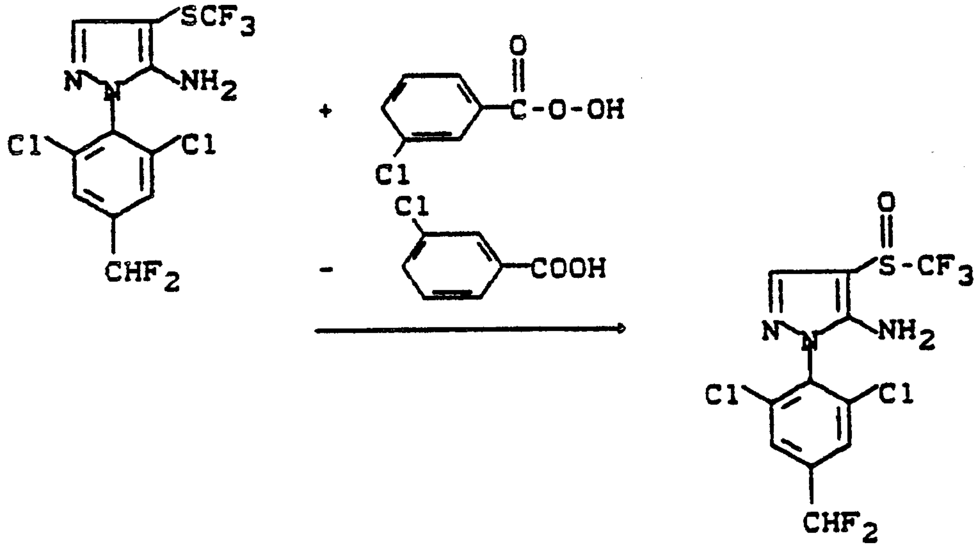

Verwendet man beispielsweise 5-Amino-1-(2,3,6-trichlor-4-difluormethylphenyl)-4-dichlorfluormethylsulfonyl-pyrazol als Ausgangsverbindungen und Natriumnitrit/Bromwasserstoffsäure als Reagenzien, so läßt sich der Reaktionsablauf des erfindungsgemäßen Verfahrensvariante (e) durch das folgende Formelschema darstellen:

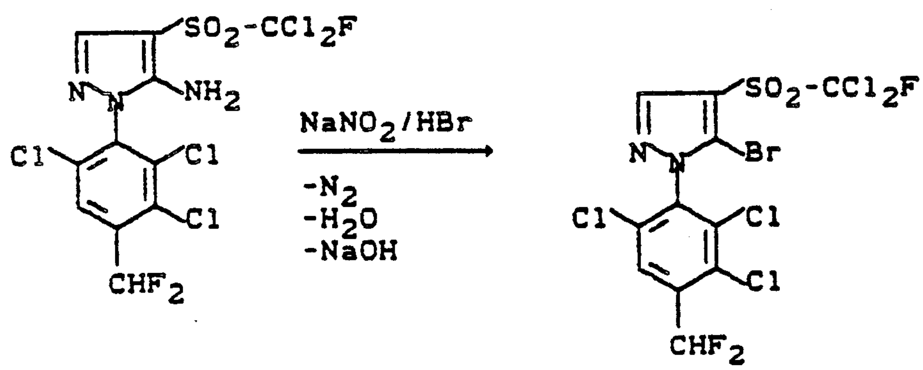

Die 4-unsubstituierten 1-Arylpyrazole der Formel (II) sind noch nicht bekannt. Die Verbindungen der Formel (II), in welchen $R^6$ für Wasserstoff, Alkyl, Alkenyl oder Alkinyl steht und $R^7$ für Alkyl, Alkenyl oder Alkinyl steht, sind Bestandteile der vorliegenden Erfindung und sollen als Verbindungen der Formel (IIb) bezeichnet werden.

Man erhält die Verbindungen der Formel (II), wenn man 1-Arylhydrazine der Formel (VI),

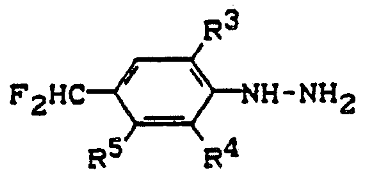

(VI)

in welcher
$R^3$, $R^4$ und $R^5$ die oben angegebene Bedeutung haben,
mit Acrylnitril der Formel (VII)

$CH_2 = CH\text{-}CN$ (VII)

gegebenenfalls in Gegenwart eines Verdünnungsmittels, beispielsweise Ethanol, bei Temperaturen zwischen 50 und 150 °C umsetzt und die so erhältlichen N-Aryl-N′-cyanethylhydrazine der Formel (VIII),

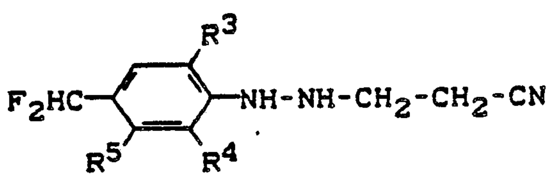

(VIII)

in welcher
$R^3$, $R^4$ und $R^5$ die oben angegebene Bedeutung haben,
in einer 2. Stufe mit Natriumhydroxid gegebenenfalls in Gegenwart eines Verdünnungsmittels, beispielsweise Ethanol, bei Temperaturen zwischen 50 °C und 120 °C umsetzt und gegebenenfalls anschließend die so erhaltenen 5-Amino-1-arylpyrazole der Formel (IIa),

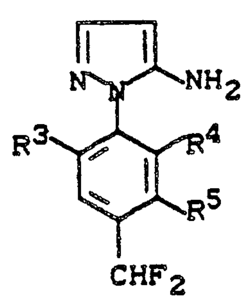

(IIa)

in welcher
$R^3$, $R^4$ und $R^5$ die oben angegebene Bedeutung haben,
entweder in Analogie zur Durchführung der erfindungsgemäßen Verfahrensvariante (c) alkyliert oder in allgemein üblicher Weise mit Acylierungsmitteln der Formel (IX),

$R^8\text{-}\underset{\underset{O}{\|}}{C}\text{-}A^2$ (IX)

in welcher
$R^8$ für Alkyl, insbesondere für Methyl oder Ethyl steht und
$A^2$ für eine elektronenanziehende Abgangsgruppe, vorzugsweise Chlor, Brom, Acetoxy oder Propionyloxy steht,
dann die so erhältlichen 5-(N-Acyl)amino-1-arylpyrazole der Formel (X),

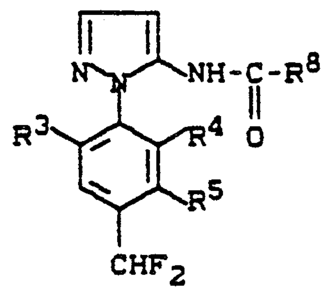

(X)

in welcher
$R^3$, $R^4$, $R^5$ und $R^8$ die oben angegebene Bedeutung haben, in Analogie zur Durchführung der erfindungsgemäßen Verfahrensvariante (c) alkyliert und schließlich die so erhältlichen 5-(N-Acylamino)-N-alkyl-1-arylpyrazole der Formel (XI),

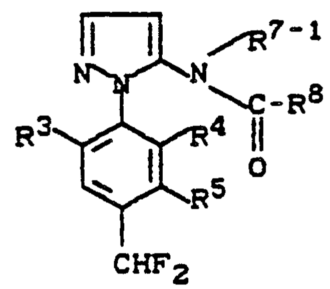

(XI)

in welcher
$R^3$, $R^4$, $R^5$ und $R^{7-1}$ und $R^8$ die oben angegebene Bedeutung haben, in Analogie zur Durchführung der erfindungsgemäßen Verfahrensvariante (b) deacyliert.

Die Arylhydrazine der Formel (VI) sind bekannt oder erhältlich in Analogie zu bekannten Verfahren (vergl. z.B. DE-OS 3 226 513; DE-OS 3 226 496 oder EP-A-224 831).

Das Acrylnitril der Formel (VII) und die Acylierungsmittel der Formel (IX) sind allgemein bekannte Verbindungen der organischen Chemie.

Die zur Durchführung des erfindungsgemäßen Verfahrens (a) weiterhin als Ausgangsstoffe benötigten Sulfenylchloride sind durch die Formel (III) allgemein definiert. In dieser Formel (III) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Sulfenylchloride der Formel (III) sind allgemein bekannte Verbindungen der organischen Chemie.

Die zur Durchführung des erfindungsgemäßen Verfahrens (b) als Ausgangsstoffe benötigten 5-(N-Acylamino)-1-arylpyrazole sind durch die Formel (IV) allgemein definiert. In dieser Formel (IV) stehen $R^1$, $R^3$, $R^4$, $R^5$ und n vorzugsweise für diejenigen Reste und Indices, die bereits im Zusammenhang mit der Beschreibung der erfindungsgemäßen Stoffe der Formel (I) als bevorzugt genannt wurden.

$R^{7-1}$ steht vorzugsweise für jeweils geradkettiges oder verzweigtes Alkyl, Alkenyl oder Alkinyl mit jeweils bis zu 6 Kohlenstoffatomen, besonders bevorzugt für Methyl, Ethyl, n- oder i-Propyl, n-, i-, s- oder t-Butyl, Allyl, Propargyl sowie jeweils geradkettiges oder verzweigtes Butenyl, Butinyl, Pentenyl oder Pentinyl, insbesondere für Methyl, Ethyl oder Allyl.

$R^8$ steht vorzugsweise für geradkettiges oder verzweigtes Alkyl mit 1 bis 4 Kohlenstoffatomen, insbesondere für Methyl oder Ethyl.

Die 5-(N-Acylamino)-1-arylpyrazole der Formel (IV) sind noch nicht bekannt.

Man erhält sie, wenn man die mit Hilfe der erfindungsgemäßen Verfahrensvariante (a) oder (d) erhältlichen 1-Arylpyrazole der Formel (Ix),

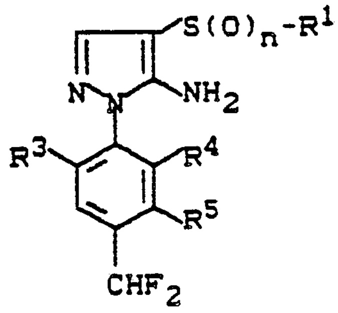

(Ix)

in welcher
$R^1$, $R^3$, $R^4$, $R^5$ und n die oben angegebene Bedeutung haben,
in üblicher Weise mit Acylierungsmitteln der Formel (IX)

$R^8$- C -$A^2$ (IX)
||
O

in welcher
$R^8$ für Alkyl, insbesondere für Methyl oder Ethyl steht und
$A^2$ für eine elektronenanziehende Abgangsgruppe, insbesondere Chlor, Brom, Acetoxy oder Propionyloxy steht,
gegebenenfalls in Gegenwart eines Verdünnungsmittels, beispielsweise Dichlormethan, und gegebenenfalls in Gegenwart eines Säurebindemittels, beispielsweise Triethylamin bei Temperaturen zwischen 0° und 80° C umsetzt und anschließend die so erhaltenen 5-(N-Acylamino)-1-arylpyrazole der Formel (XII),

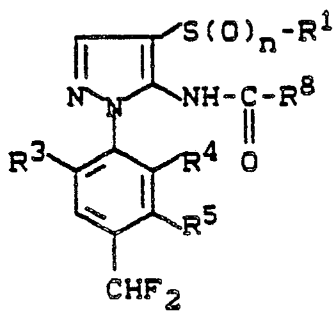

(XII)

in welcher
$R^1$, $R^3$, $R^4$, $R^5$, $R^8$ und n die oben angegebene Bedeutung haben
in Analogie der Durchführung der erfindungsgemäßen Verfahrensvariante (c) alkyliert.

Die 1-Arylpyrazole der Formel (Iz) gehören zu den erfindungsgemäßen Verbindungen und sind mit Hilfe der erfindungsgemäßen Verfahrensvarianten (a), (b) oder (d) erhältlich.

Die Alkylierungsmittel der Formel (V) sind allgemein bekannte Verbindungen der organischen Chemie.

In den Verbindungen der Formel (V) steht $A^1$ vorzugsweise für Halogen oder für gegebenenfalls substituiertes Alkoxysulfonyloxy oder Arylsulfonyloxy, insbesondere für Chlor, Brom oder lod oder für Methoxysulfonyloxy, Ethoxysulfonyloxy oder p-Toluolsulfonyloxy.

Die zur Durchführung der erfindungsgemäßen Verfahrensvariante (d) als Ausgangsstoffe benötigten 1-Arylpyrazole der Formel (Ia) gehören zu den erfindungsgemäßen Verbindungen und sind mit Hilfe der erfindungsgemäßen Verfahrensvarianten (a), (b) oder (c) erhältlich.

Die zur Durchführung des erfindungsgemäßen Verfahrens (e) als Ausgangsstoffe benötigten 1-Arylpyrazole der Formel (Iy) gehören ebenfalls zu den erfindungsgemäßen Verbindungen und sind mit Hilfe der erfindungsgemäßen Verfahren (a) und (d) erhältlich.

Als Verdünnungsmittel zur Durchführung des erfindungsgemäßen Verfahrens (a) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile,

wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Säuren wie beispielsweise Essigsäure.

Das erfindungsgemäße Verfahren (a) wird gegebenenfalls in Gegenwart eines Säurebindemittels durchgeführt. Als solche kommen alle üblichen anorganischen oder organischen Base infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen(DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -20 °C und +120 °C, vorzugsweise bei Temperaturen zwischen 0 °C und +50 °C.

Zur Durchführung des erfindungsgemäßen Verfahrens (a) setzt man pro Mol an 4-unsubstituierten 1-Aryl-pyrazol der Formel (II) im allgemeinen 1,0 bis 2,5 Mol, vorzugsweise 1,0 bis 1,5 Mol an Sulfenylchlorid der Formel (III) und 1,0 bis 2,5 Mol, vorzugsweise 1,0 bis 1,5 Mol an Säurebindemittel ein. Die Reaktionsführung, Aufarbeitung und Isolierung der Reaktionsprodukte der Formel (Ia) erfolgt in allgemein üblicher Weise.

Als Verdünnungsmittel zur Durchführung des erfindungsgemäßen Verfahrens (b) kommen anorganische oder organische polare Lösungsmittel in Frage. Vorzugsweise verwendet man Alkohole, wie beispielsweise Methanol, Ethanol oder Propanol, oder deren Gemische mit Wasser.

Als Katalysatoren zur Durchführung des erfindungsgemäßen Verfahrens (b) kommen vorzugsweise anorganische Mineralsäuren, insbesondere Chlorwasserstoffsäure oder Schwefelsäure infrage.

Die Reaktionstemperaturen können bei der Durchführung des erfindungsgemäßen Verfahrens (b) in einem größeren Bereich variiert werden. Im allgemeinen arbeitet man zwischen +20 °C und +150 °C, vorzugsweise zwischen +50 °C und +120 °C.

Zur Durchführung des erfindungsgemäßen Verfahrens (b) setzt man pro Mol 5-(N-Acylamino)-1-arylpyrazol der Formel (IV) im allgemeinen 1,0 bis 20,0 Mol, vorzugsweise 1,0 bis 10,0 Mol an Katalysatorsäure ein und erwärmt für mehrere Stunden auf die erforderliche Reaktionstemperatur. Die Aufarbeitung, Isolierung und Reinigung der Reaktionsprodukte der Formel (Ib) erfolgt nach üblichen Methoden.

Als Verdünnungsmittel zur Durchführung des erfindungsgemäßen Verfahrens (c) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (c) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethylammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Reaktionshilfsmittel zur Durchführung des erfindungsgemäßen Verfahrens (c) kommen alle üblicherweise verwendbaren anorganischen und organischen Basen infrage. Vorzugsweise verwendet man Alkalimetallhydride, -hydroxide, -amide, -alkholate, -carbonate oder hydrogencarbonate, wie beispielsweise Natriumhydrid, Natriumamid, Natriummethylat, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (c) setzt man pro Mol an 1-Arylpyrazol der Formel (Iz) im allgemeinen 1,0 bis 20,0 Mol, vorzugsweise 1,0 bis 15,0 Mol an Alkylierungsmittel der Formel (V) und gegebenenfalls 1,0 bis 3,0 Mol, vorzugsweise 1,0 bis 2,0 Mol an Reaktionshilfsmittel sowie gegebenenfalls 0,01 bis 1,0 Mol an Phasentransferkatalysator ein. Die Reaktionsdurchführung, Aufarbeitung und Isolierung der Reaktionsprodukte der Formel (Ic) erfolgt in allgemein üblicher Weise.

Als Oxidationsmittel zur Durchführung des erfindungsgemäßen Verfahrens (d) kann man alle üblicherweise für Schwefeloxidationen infrage kommenden anorganischen oder organischen Oxidationsmittel verwenden. Vorzugsweise verwendet man organische Persäuren, wie beispielsweise Peressigsäure, 4-Nitroperbenzoesäure oder 3-Chlorperbenzoesäure, anorganische Persäuren, wie beispielsweise Periodsäure oder auch Wasserstoffperoxid, Kaliumpermanganat oder Chromsäure.

Als Verdünnungsmittel zur Durchführung des erfindungsgemäßen Verfahrens (d) kommen ebenfalls inerte organische Lösungsmittel infrage.
Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (d) kann gegebenenfalls in Gegenwart eines Reaktionshilfsmittels, insbesondere eines Säurebindemittels durchgeführt werden. Als solche kommen alle üblicherweise verwendbaren organischen und anorganischen Säurebindemittel infrage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (d) kann gegebenenfalls in Gegenwart eines geeigneten Katalysators durchgeführt werden. Als solche kommen alle üblicherweise für derartige Schwefeloxidationen üblichen Katalysatoren infrage. Beispielhaft genannt seien in diesem Zusammenhang Schwermetallkatalysatoren wie Ammoniummolybdat.

Die Reaktionstemperaturen können bei der Durchführung des erfindungsgemäßen Verfahrens (d) in einem größeren Bereich variiert werden. Im allgemeinen arbeitet man bei Temperaturen zwischen -20° C und +70° C, vorzugsweise bei Temperaturen zwischen 0° C und +50° C.

Zur Durchführung des erfindungsgemäßen Verfahrens (d) setzt man pro Mol an 1-Aryl-pyrazol der Formel (Ia) im allgemeinen 0,8 bis 1,2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an 1-Aryl-pyrazol der Formel (Ia) im allgemeinen 1,8 bis 3,0 Mol, vorzugsweise doppelt molare Mengen an Oxidationsmittel ein. Die Reaktionsführung, Aufarbeitung und Isolierung der Endprodukte der Formel (Id) erfolgt nach üblichen Verfahren.

Als Verdünnungsmittel zur Durchführung des erfindungsgemäßen Verfahrens (e) kommen alle üblicherweise für derartige Diazotierungsreaktionen üblichen Lösungsmittel in Frage. Vorzugsweise verwendet man Halogenkohlenwas serstoffe wie Chloroform oder Bromoform oder wässrige Säuren wie bei spielsweise Halogenwasserstoffsäuren oder Schwefelsäure, wobei die Säurekomponente gleichzeitig als Reagenz und/oder als Reaktionshilfsmittel fungiert. Bei der Verwendung von Bromoform als Verdünnungsmittel erhält man in der Regel die entsprechenden 5-Brom-pyrazole der Formel (Ie), wobei das Bromoform gleichzeitig als Verdünnungsmittel und als Reagenz fungiert.
Die entsprechende Reaktion in Gegenwart von Chloroform als Verdünnungsmittel ergibt im allgemeinen eine Mischung von 5-Chlor-pyrazolverbindungen der Formel (Ie) und den analogen reduzierten Verbindungen der Formel (Ie) die in der 5-Position des Pyrazolringes einen Wasserstoffrest tragen. Diese Mischungen lassen sich destillativ auftrennen.

Das erfindungsgemäße Verfahren (e) wird in Gegenwart eines anorganischen oder organischen Nitrits durchgeführt. Als solche kommen alle üblicherweise für derartige Diazotierungsreaktionen üblichen Nitritverbindungen in Frage. Besonders bevorzugt verwendet man Alkalimetallnitrite, wie beispielsweise Natriumnitrit oder Alkylnitrite wie beispielsweise t-Butylnitrit.

Das erfindungsgemäße Verfahren (e) wird gegebenenfalls in Gegenwart einer Halogenwasserstoffsäure durchgeführt. In diesem Fall erhält man als Reaktionsprodukte 1-Aryl-pyrazole der Formel (Ie), bei welchen der Rest $R^{2-1}$ für einen Halogenrest steht, der dem Anion der verwendeten Halogenwasserstoffsäure entspricht. Vorzugsweise verwender man jeweils wässrige Lösungen der Fluorwasserstoffsäure, Chlorwasserstoffsäure, Bromwasserstoffsäure oder Iodwasserstoffsäure.

Das erfindungsgemäße Verfahren (e) wird üblicherweise in Gegenwart eines Reaktionshilfsmittels durchgeführt. Als solche kommen insbesondere starke Mineralsäuren wie Schwefelsäure oder Phosphorsäure oder die oben aufgeführten Halogenwasserstoffsäuren in Frage, die in diesem Fall gleichzeitig als Reagenz und als Katalysator wirken.

Das erfindungsgemäße Verfahren (e) kann gegebenenfalls in Gegenwart eines geeigneten Reduktionsmittels durchgeführt werden. In diesem Fall erhält man als Reaktionsprodukte 1-Aryl-pyrazole der Formel (Ie), bei welchen de Rest $R^{2-1}$ für Wasserstoff steht. Als Reduktionsmittel verwendet man in diesen Fällen besonders bevorzugt unterphosphorige Säure ($H_3PO_2$).

Die Reaktionstemperaturen können bei der Durchführung des erfindungsgemäßen Verfahrens (e) in einem größeren Bereich variiert werden. Im allgemeinen arbeitet man bei Temperaturen zwischen -30° C und +60° C, vorzugsweise bei Temperaturen zwischen -20° C und +40° C.

Zur Durchführung des erfindungsgemäßen Verfahrens (e) setzt man pro Mol an 1-Arylpyrazol der Formel (Iy) im allgemeinen 1,0 bis 1,8 Mol an Nitrit, gegebenenfalls 1,0 bis 20,0 Mol, vorzugsweise 1,0 bis 10,0 Mol an Halogenwasserstoffsäure, gegebenenfalls 1,0 bis 50,0 Mol, vorzugsweise 1,0 bis 20,0 Mol an Reduktionsmittel und gegebenenfalls 1,0 bis 20,0 Mol, vorzugsweise 1,0 bis 10,0 Mol an als Reaktionshilfsmittel verwendete Mineralsäure ein.

Dabei setzt man üblicherweise der Reaktionsmischung bestehend aus 1-Arylpyrazol der Formel (Iy), Mineralsäure, Verdünnungsmittel und Halogenwasserstoffsäure bzw. Reduktionsmittel das Nitrit in kleinen Portionen gegebenenfalls in geeignetem Verdünnungsmittel gelöst zu.

Die Aufarbeitung und Isolierung der Reaktionsprodukte der Formel (Ie) erfolgt nach üblichen Methoden z.B. durch Abfiltrieren von kristallinen Produkten oder durch Extraktion mit einem geeigneten organischen Lösungsmittel. Die Identifizierung erfolgt durch Schmelzpunkt oder Protonen-Kernresonanz-Spektrum.

Erfindungsgemäße Verbindungen der Formel (Ie1)) ($R^2$ der Formel (I) bedeutet Wasserstoff)

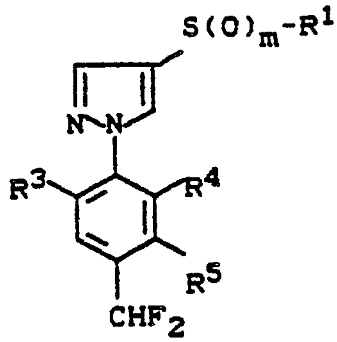

(Ie1)

in welcher
$R^1$, $R^3$, $R^4$, und $R^5$ die oben angegebene Bedeutung haben und
m für die Zahl 1 oder 2 steht,
erhält man alternativ auch aus den zu den erfindungsgemäßen Verbindungen gehörenden Verbindungen der Formel (Ie2),

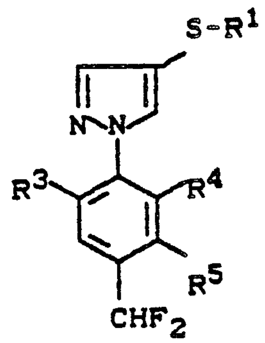

(Ie2)

in welcher
$R^1$, $R^3$, $R^4$, und $R^5$ die oben angegebene Bedeutung haben,
wenn man Analogie zur Durchführung des erfindungsgemäßen Verfahrens (d) mit Oxidationsmitteln gegebenenfalls in Gegenwart eines Verdünnungsmittels wie beispielsweise Dichlormethan und gegebenenfalls in Gegenwart eines Katalysators wie beispielsweise Ammoniummolybdat und gegebenenfalls in Gegenwart eines Säurebindemittels wie beispielsweise Natriumcarbonat oder Natriumbicarbonat bei Temperaturen zwischen 0° C und 50° C am Schwefel der Sulfenylgruppe in 4-Position des Pyrazolringes oxidiert.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats- und Materialschutz sowie auf dem Hygienesektor vorkommen. Sie sind gegen

normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Chloristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räubemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten und Endoparasiten wirksam.

Die erfindungsgemäßen Wirkstoffe der Formel (I) zeichnen sich durch eine hohe insektizide Wirksamkeit aus. Sie lassen sich insbesondere gegen pflanzenschädigende Insekten, wie beispielsweise gegen die schwarze Bohnenblattlaus (Aphis fabae) oder gegen die Larven der Meerrettichblattkäfer (Phaedon cochleariae), einsetzen. Dabei zeigen die erfindungsgemäßen Wirkstoffe auch blattsystemische Eigenschaften. Sie eignen sich daneben auch zur Bekämpfung von Bodeninsekten.

Außerdem besitzen die erfindungsgemäßen Wirkstoffe der Formel (I) eine hohe Wirkung gegen Hygiene- und Vorratsschädlinge und lassen sich beispielsweise zur Bekämpfung der deutschen Schabe (Blattella germanica) einsetzen.

Darüber hinaus lassen sich die erfindungsgemäßen Wirkstoffe mit besonders gutem Erfolg zur Bekämpfung von von parasitisch lebenden Warmblüterschädlingen, wie beispielsweise gegen endoparasitisch lebende Nematoden der Gattung Caenorhabditis elegans einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den

aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise und in den üblichen Formulierungen wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Im vorliegenden Text beziehen sich alle Prozentangaben auf Gewichtsprozente, wo nichts anderes angegeben ist.

Das erfindungsgemäße Herstellungsverfahren sowie die biologische Wirksamkeit der erfindungsgemäßen Verbindungen sollen anhand der folgenden Beispiele erläutert werden:

Herstellungsbeispiele:

Beispiel 1:

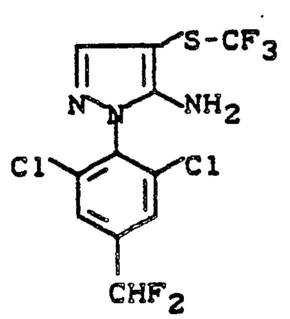

(Verfahrensvariante (a))

In eine Lösung von 14 g (0,053 Mol) 5-Amino-1-(2,6-dichlor-4-difluormethyl-phenyl)-pyrazol in 80 ml Eisessig leitet man bei ca. 15 °C 8,6 g (0,063 Mol) Trifluormethansulfenylchlorid. Danach rührt man 1 Stunde bei Raumtemperatur, gibt dann die Reaktionsmischung in 500 ml Wasser, saugt den so erhaltenen kristallinen Niederschlag ab und trocknet ihn im Vakuum. Man erhält 18,5 g (92% der Theorie) an 5-Amino-1-(2,6-dichlor-4-difluormethyl-phenyl)-4-trifluormethylthiopyrazol vom Schmelzpunkt 117-118 °C.

Herstellung der Ausgangsverbindungen:

Beispiel II-1:

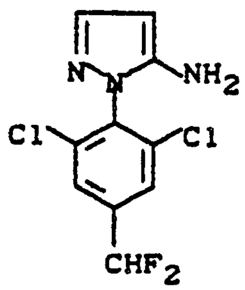

29,8 g (0,1 Mol) N-(2,6-Dichlor-4-difluormethylphenyl)-N′-(2-cyanethyl)-hydrazin in 200 ml Ethanol werden mit 18 g (0,2 Mol) 45%iger technischer Natronlauge etwa 2 Stunden auf Rückflußtemperatur erhitzt. Nach beendeter Reaktion (dünnschichtchromatographische Kontrolle; Dichlormethan/Methanol 19:1) engt man im Vakuum ein, nimmt den Rückstand in Dichlormethan/Wasser auf, trennt die organische Phase ab, wäscht mit Wasser, trocknet über Natriumsulfat und entfernt das Lösungsmittel im Vakuum. Nach Umkristallisation aus 500 ml Toluol erhält man 16 g (58% der Theorie) an 5-Amino-1-(2,6-dichlor-4-difluormethylphenyl)-pyrazol vom Schmelzpunkt 110°-111° C.

$^{19}$F-NMR ($CDCl_3$): $\delta$ = 34,3 (d, 2F) ppm.

Beispiel III-1:

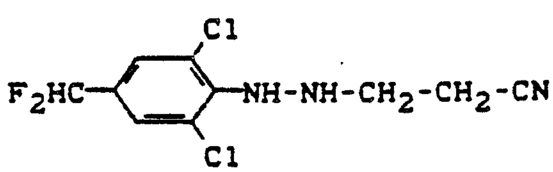

24,5 g (0,1 Mol) 2,6-Dichlor-4-difluormethylphenylhydrazin und 6 g (0,11 Mol) Acrylnitril werden in 100 ml Ethanol 4 Tage auf Rückflußtemperatur erhitzt. Nach Entfernen der flüchtigen Bestandteile im Vakuum erhält man 30 g (99% der Theorie) an N-(2,6-Dichlor-4-difluormethylphenyl)-N-(2-cyanethyl)hydrazin vom Schmelzpunkt < 45° C.

$^{1}$H-NMR ($CDCl_3$/TMS ):
$\delta$ = 2.55 (2H);
3,1 (2H);
4,55 (1H); 6,0 (1H); 7,55 (2H) ppm.

Beispiel 2:

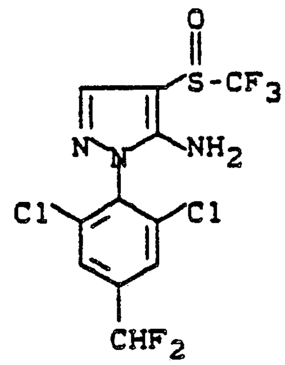

(Verfahrensvariante (d))

4 g (0,01 Mol) 5-Amino-1-(2,6-dichlor-4-difluormethylphenyl)-4-trifluormethylthiopyrazol werden bei Raumtemperatur in 20 ml 80%iger Schwefelsäure gelöst und bei ca. 15° C mit 1 ml (0,012 Mol) 35%igem Wasserstoffperoxid versetzt. Die Reaktionsmischung wird 1 Stunde bei 15° bis 20° gerührt und danach zu 300 ml Wasser gegeben. Der kristalline Niederschlag wird abfiltriert, gewaschen und im Vakuum getrocknet.

Man erhält 3,5 g (84% der Theorie) an 5-Amino-1-(2,6-dichlor-4-difluormethyl-phenyl)-4-trifluormethylsulfinyl-pyrazol vom Schmelzpunkt 60-65° C.

Beispiel 3:

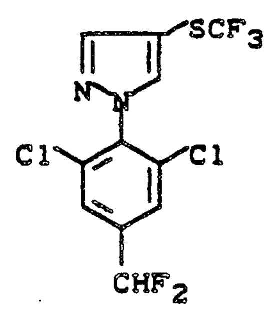

(Verfahrensvariante (e))

5 g (0,013 Mol) 5-Amino-1-(2,6-dichlor-4-difluormethylphenyl)-4-trifluormethylthiopyrazol werden in 35 ml Tetrahydrofuran gelöst, mit 3,5 ml (0,0264 Mol) Pentylnitrit versetzt und 2 Stunden auf Rückflußtemperatur erwärmt. Danach wird das Lösungsmittel im Vakuum entfernt und der ölige Rückstand rektifiziert.

Man erhält 4,2 g (87% der Theorie) 1-(2,6-Dichlor-4-difluormethyl-phenyl)-4-trifluormethylthiopyrazol vom Siedepunkt 125°-138° C bei 0,5 mbar und vom Brechungsindex $n_D^{20}$ = 1,5119.

In entsprechender Weise und gemäß den obigen allgemeinen Angaben zur Herstellung erhält man die folgenden 1-Arylpyrazole der allgemeinen Formel (I):

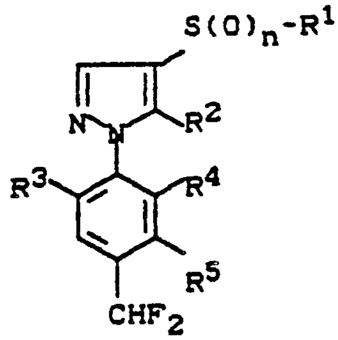

(I)

| Bsp. Nr. | $R^1-S(O)_n-$ | $R^2$ | $R^3$ | $R^4$ | $R^5$ | Schmelzpunkt /° C |
|---|---|---|---|---|---|---|
| 4 | $FCl_2C-S-$ | $-NH_2$ | Cl | Cl | H | 77-79 |
| 5 | $FCl_2C-\overset{O}{\overset{\|}{S}}-$ | $-NH_2$ | Cl | Cl | H | 78-85 |
| 6 | $F_3C-SO_2-$ | $-NH_2$ | Cl | Cl | H | 119 |
| 7 | $FCl_2C-SO_2-$ | $-NH_2$ | Cl | Cl | H | 70-75 |

Beispiel A

Phaedon-Larven-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigten z.B. die Verbindungen der Herstellungsbeispiele 4,5 und 7 bei einer beispielhaften Konzentration von 0,0001% nach 3 Tagen eine Abtötung von 100%.

Beispiel B

Aphis-Test (systemische Wirkung)

Lösungsmittel: 7 Gewichtsteile

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigte z.B. die Verbindungen des Herstellungsbeispiels 1 bei einer beispielhaften Konzentration von 0,1% nach 4 Tagen eine Abtötung von 100%.

## Beispiel C

$LD_{100}$-Test

Testtiere: Blattella germanica

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man etwa 10 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %.

Bei diesem Test zeigten z.B. die Verbindungen der Herstellungsbeispiele 1 und 3 bei einer beispielhaften Wirkstoffkonzentration von 0,02% eine Abtötung von 100%.

## Beispiel D

In vitro Nematodentest

Caenorhabditis elegans

$10^{-4}$ g Wirkstoff werden in 1 ml Wasser oder 0,1 ml Dimethylsulfoxid (DMSO) gelöst. Diese Lösung wird auf eine Replica-Platte gegeben. Dazu gibt man 2 ml einer E.coli-Suspension, zu der man 10-20 weibliche Tiere oder Larven von Caenorhabditis elegans in 0,5 ml steriler M9 Pufferlösung gegeben hat. Die E.coli-Suspension wird hergestellt indem man 300 ml einer Übernachtkultur eines Uracil-bedürftigen E.coli-Stammes mit 1,8 l steriler M9 Pufferlösung versetzt.

Der Versuchsansatz wird 7 Tage bei 22 °C inkubiert und danach ausgewertet. Es wird bewertet inwieweit der Wirkstoff die Vermehrung beeinträchtigt und die Konzentration angegeben bei der die

Vermehrung verhindert wird.

Bei diesem Test zeigten z.B. die Verbindungen der Herstellungsbeispiele 3 und 7 bei einer beispielhaften Konzentration von 100 µg/ml eine mindestens 95%ige Hemmung der Vermehrung des Nematoden C. elegans.

## Ansprüche

1. 1-Arylpyrazole der allgemeinen Formel (I),

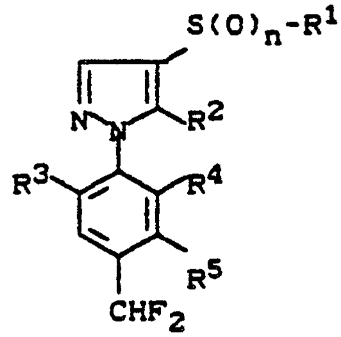

(I)

in welcher
$R^1$ für Alkyl oder Halogenalkyl steht,
$R^2$ für Wasserstoff, Halogen oder für einen Rest

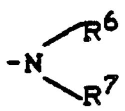

steht, in welchem
$R^6$ und $R^7$ unabhängig voneinander für Wasserstoff, Alkyl, Alkenyl oder Alkinyl stehen,
$R^3$ für Wasserstoff oder Halogen steht,
$R^4$ für Halogen steht,
$R^5$ für Wasserstoff oder Halogen steht und
n für eine Zahl 0, 1 oder 2 steht.

2. 1-Arylpyrazole der Formel (I) gemäß Anspruch 1, in welchen
$R^1$ für Alkyl mit 1 bis 4 Kohlenstoffatomen oder für Halogenalkyl mit 1 bis 4 Kohlenstoffatomen und 1 bis 9 gleichen oder verschiedenen Halogenatomen steht,
$R^2$ für Wasserstoff, Fluor, Chlor, Brom oder für einen Rest

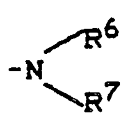

steht, in welchem
$R^6$ und $R^7$ unabhängig voneinander für Wasserstoff, Alkyl mit 1 bis 6 Kohlenstoffatomen, Alkenyl oder Alkinyl mit 2 bis 6 Kohlenstoffatomen stehen,
$R^3$ für Wasserstoff, Fluor, Chlor oder Brom steht,
$R^4$ für Fluor, Chlor oder Brom steht,
$R^5$ für Wasserstoff, Fluor, Chlor oder Brom steht und
n für eine Zahl 0, 1 oder 2 steht.

3. 1-Arylpyrazole der Formel (I) gemäß Anspruch 1, in welchen
$R^1$ für Halogenalkyl mit 1 bis 4 Kohlenstoffatomen und1 bis 9 gleichen oder verschiedenen Halogenatomen steht,
$R^2$ für Wasserstoff oder die $-NH_2$-Gruppe steht,
$R^3$ und $R^4$ für Halogen stehen,
$R^5$ für Wasserstoff steht und
n 0, 1 oder 2 bedeutet.

4. 1-Arylpyrazole der Formel (I) gemäß Anspruch 1, in welcher
$R^1$ für Trifluormethyl oder Dichlorfluormethyl steht,
$R^2$ für Wasserstoff oder $-NH_2$ steht,
$R^3$ und $R^4$ für Chlor stehen,
$R^5$ für Wasserstoff steht; und
n für 0, 1 oder 2 steht.

5. Verfahren zur Herstellung der 1-Arylpyrazole der allgemeinen Formel (I),

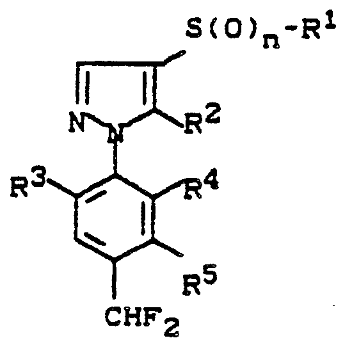

(I)

in welcher
$R^1$ für Alkyl oder Halogenalkyl steht,
$R^2$ für Wasserstoff, Halogen oder für einen Rest

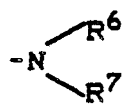

steht, in welchem
$R^6$ und $R^7$ unabhängig voneinander für Wasserstoff-, Alkyl, Alkenyl oder Alkinyl stehen,
$R^3$ für Wasserstoff oder Halogen steht,
$R^4$ für Halogen steht,
$R^5$ für Wasserstoff- oder Halogen steht und
n für eine Zahl 0, 1 oder 2 steht,
dadurch gekennzeichnet, daß man

(a) zur Herstellung der 1-Arylpyrazole der Formel (Ia),

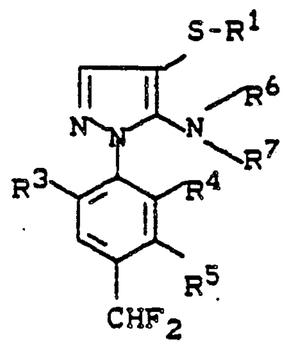

(Ia)

in welcher

$R^1$, $R^3$, $R^4$, $R^5$, $R^6$ und $R^7$ die oben angegebene Bedeutung haben,

4-unsubstituierte 1-Arylpyrazole der Formel (II),

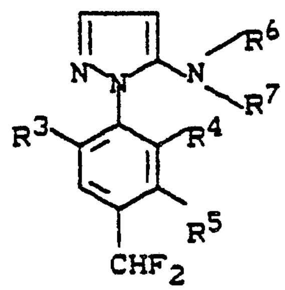

(II)

in welcher

$R^3$, $R^4$, $R^5$, $R^6$ und $R^7$ die oben angegebene Bedeutung haben,

mit Sulfenylchloriden der Formel (III),

$Cl-S-R^1$ (III)

in welcher

$R^1$ die oben angegebene Bedeutung hat,

gegebenenfalls in Gegenwart eines Verdünnungsmittels und gegebenenfalls in Gegenwart eines Säurebindemittels umsetzt; oder

b) zur Herstellung der 1-Arylpyrazole der Formel (Ib),

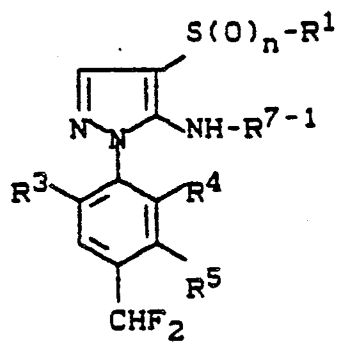

(Ib)

in welcher

$R^1$, $R^3$, $R^4$, $R^5$ und n die oben angegebene Bedeutung haben und

$R^{7-1}$ für Alkyl, Alkenyl oder Alkinyl steht,

5-(N-Acylamino)-1-arylpyrazole der Formel (IV),

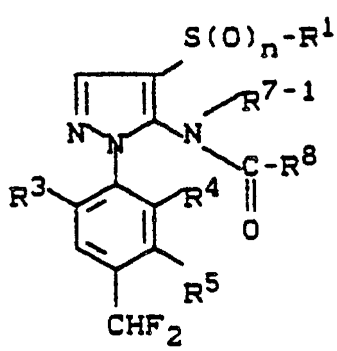

(IV)

in welcher

$R^1$, $R^3$, $R^4$, $R^5$, $R^{7-1}$ und n die oben angegebene Bedeutung haben und
$R^8$ für Alkyl steht,
mit Säuren als Katalysator gegebenenfalls in Gegenwart eines Verdünnungsmittels deacyliert; oder
(c) zur Herstellung der 1-Arylpyrazole der Formel (Ic),

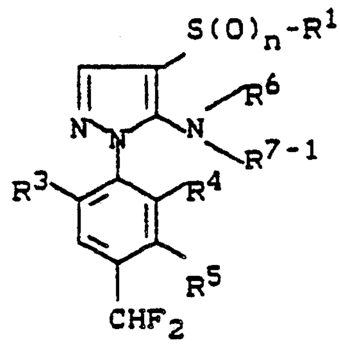

(Ic)

in welcher
$R^1$, $R^3$, $R^4$, $R^5$, $R^6$ und n die oben angegebene Bedeutung haben und
$R^{7-1}$ für Alkyl, Alkenyl oder Alkinyl steht,
1-Arylpyrazole der Formel (Iz)

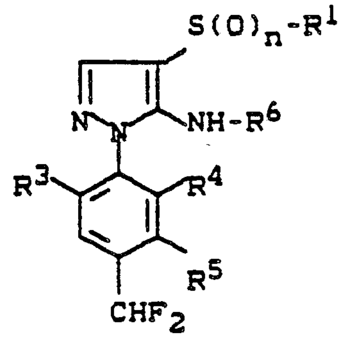

(Iz)

in welcher
$R^1$, $R^3$, $R^4$, $R^5$, $R^6$ und n die oben angegebene Bedeutung haben,
mit Alkylierungsmitteln der Formel (V),

$R^{7-1}$ - $A^1$   (V)

in welcher
$R^{7-1}$ die oben angegebene Bedeutung hat und
$A^1$ für eine elektronenanziehende Abgangsgruppe steht,
gegebenenfalls in Gegenwart eines Verdünnungsmittels und gegebenenfalls in Gegenwart eines Reaktionshilfsmittels umsetzt; oder
(d) zur Herstellung der 1-Arylpyrazole der Formel (Id),

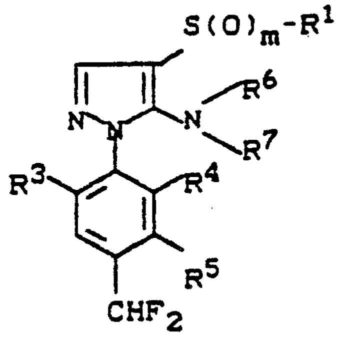

(Id)

in welcher
$R^1$, $R^3$, $R^4$, $R^5$, $R^6$ und $R^7$ die oben angegebene Bedeutung haben und
m für eine Zahl 1 oder 2 steht,
1-Arylpyrazole der Formel (Ia),

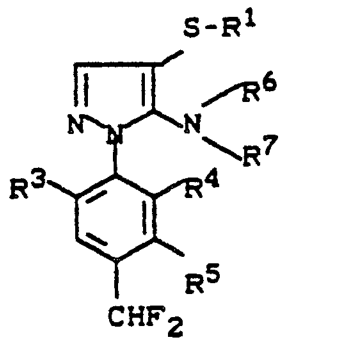

(Ia)

$R^1$, $R^3$, $R^4$, $R^5$, $R^6$ und $R^7$ die oben angegebene Bedeutung haben,
mit einem Oxidationsmittel, gegebenenfalls in Gegenwart eines Verdünnungsmittels, gegebenenfalls in Gegenwart eines Reaktionshilfsmittels und gegebenenfalls in Gegenwart eines Katalysators umsetzt; oder

(e) zur Herstellung der 1-Arylpyrazole der Formel (Ie),

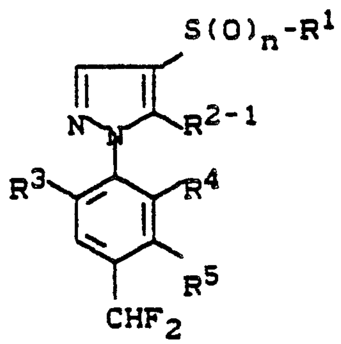

(Ie)

in welcher
$R^1$, $R^3$, $R^4$, $R^5$ und n die oben angegebene Bedeutung haben und
$R^{2-1}$ für Wasserstoff oder Halogen steht,
1-Arylpyrazole der Formel (Iy),

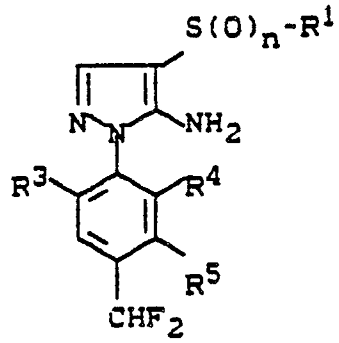

(Iy)

in welcher
$R^1$, $R^3$, $R^4$, $R^5$ und n die oben angegebene Bedeutung haben,
mit einem anorganischem order organischen Nitrit in Gegenwart eines Reaktionshilfsmittels und gegebenenfalls in Gegenwart einer Halogenwasserstoffsäure sowie gegebenenfalls in Gegenwart eines Verdünnungsmittels umsetzt.

6. Schädlingsbekämpfungsmittel, gekennzeichnet durch einen Gehalt an mindestens einer Verbindung der allgemeinen Formel (I) gemäß Anspruch 1 oder 5.

7. Verwendung von Verbindungen der allgemeinen Formel (I) gemäß Anspruch 1 oder 5 zur Bekämpfung von Schädlingen, vorzugsweise Arthropoden, insbesondere Insekten und Nematoden.

8. Verfahren zur Bekämpfung von Schädlingen, dadurch gekennzeichnet, daß man Verbindungen der allgemeinen Formel (I) gemäß Anspruch 1 oder 5 auf Schädlinge, vorzugsweise Arthropoden, insbesondere Insekten oder Nematoden oder ihren Lebensraum einwirken läßt.

9. Verfahren zur Herstellung von Schädlingsbekämpfungsmitteln, dadurch gekennzeichnet, daß man Verbindungen der allgemeinen Formel (I) gemäß Anspruch 1 cder 5 mit Streckmitteln und/oder oberflächenaktiven Mitteln vermischt.

10. 1-Arylpyrazole der Formel (IIb)

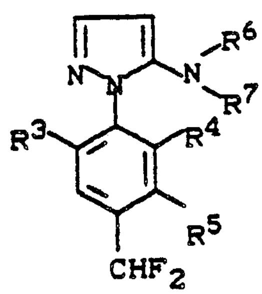

(IIb)

in welcher
$R^3$ für Wasserstoff oder Halogen steht,
$R^4$ für Halogen steht,
$R^5$ für Wasserstoff oder Halogen steht,
$R^6$ für Wasserstoff, Alkyl, Alkenyl oder Alkinyl steht und
$R^7$ für Alkyl, Alkenyl oder Alkinyl steht.

11. 1-Arylpyrazole der Formel (IV)

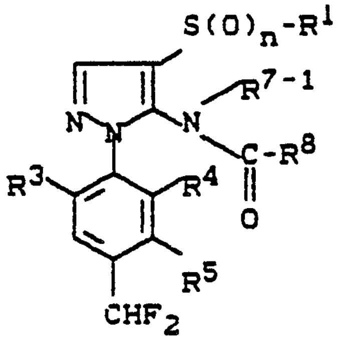

(IV)

in welcher
$R^1$ für Alkyl oder Halogenalkyl steht,
$R^3$ für Wasserstoff oder Halogen steht,
$R^4$ für Halogen steht,
$R^5$ für Wasserstoff oder Halogen steht,
$R^{7-1}$ für Alkyl, Alkenyl oder Alkinyl steht,
$R^8$ für Alkyl steht und
n für 0, 1 oder 2 steht.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 88112697.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP - A2 - 0 235 628 (BAYER AG) * Ansprüche 1,4-9; Seite 16, Zeile 4 * | 1,5,6-11 | C 07 D 231/44<br>C 07 D 231/22<br>A 01 N 43/56 |
| A | DE - A1 - 3 529 829 (BAYER AG) * Ansprüche 1,4-9; Seite 2, Zeilen 60,61; Seite 3, Formeln I,II * | 1,5,6-11 | |

RECHERCHIERTE SACHGEBIETE (Int. Cl.4)

C 07 D 231/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6,9-11

Unvollständig recherchierte Patentansprüche: 7,8

Nicht recherchierte Patentansprüche: –

Grund für die Beschränkung der Recherche:

(Verfahren zur therapeutischen Behandlung des menschlichen oder tierischen Körpers. Art. 52(4), EPÜ)

| Recherchenort | Abschlußdatum der Recherche | Prüfer |
|---|---|---|
| WIEN | 14-11-1988 | BRUS |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument